# EUROPEAN PATENT APPLICATION

(11) **EP 3 766 475 A1**
(43) Date of publication of application: **20.01.2021**
(21) Application number: 20185125.0
(22) Date of filing: 10.07.2020
(51) Int. Cl.: A61K 8/02, A61K 8/34, A61K 8/73, A61Q 19/10

(54) **WIPES**

(30) Priority: 15.07.2019 US 201962874210 P
(71) Applicant: OP-Hygiene IP GmbH, 4704 Niederbipp (CH)
(72) Inventor: OPHARDT, Heiner, 4422 Arisdorf (CH)
(74) Representative: Grünecker Patent- und Rechtsanwälte PartG mbB

(57) **Abstract**

A wipe comprising a substrate and a cleaning and/or disinfecting compound retained by one or more cyclodextrin compounds. The wipe is wet with one or more fluids comprising water to release the cleaning and/or disinfecting compound from the one or more cyclodextrin compounds, and applied to a surface to clean and/or disinfect the surface.

## Description

### Scope of the Invention

This invention relates to wipes and more particularly to wipes including a substrate preferably sheet-like to be used for cleaning surfaces and which substrate carries or is impregnated with cyclodextrin compounds that retain a cleaning and/or disinfecting compound preferably with a propensity for killing pathogens, such as ethanol, with the disinfecting compounds being released on the wetting of the wipes with water.

The applicant has appreciated the disadvantage that after a person has cleaned their hands, the person in drying their hands often exposes the hands to contamination. Such contamination arises, for example, when an air blowing hand dryer may be used as, for example, in the user contacting the sides or surfaces of the air blower.

The applicants have appreciated the use of air dryers to dry a person's hands which become wetted with the fluid during the cleaning operation and, as well, the use of alcohol cleaners in which the cleaning fluid is to evaporate from a person's hands without the need for drying have the disadvantages that the mechanical engagement of the surface of the hands as with a drying sheet is not carried out with the disadvantage that there is no mechanical engagement of a drying sheet that can assist in removing contaminants from the hands.

### Background of the Invention

Cyclodextrins are supramolecular oligosaccharides, having five or more α-d-glucopyranoside units linked by α-1,4-glycosidic bonds in a ring shape. The frequently used Cyclodextrins have 6, 7, or 8 glucopyranoside units and are called α-cyclodextrin, β-cyclodextrin, or γ-cyclodextrin, respectively. Cyclodextrins have toroid cone structures resulting into hydrophobic core and hydrophilic exterior. The interior of the cyclodextrin molecule is a chiral, nonpolar, hydrophobic cavity.

With a hydrophobic interior and hydrophilic exterior, Cyclodextrins form complexes with hydrophobic compounds that readily forms stable inclusion complexes with a wide range of chemical substances. Such molecular inclusion complexes that Cyclodextrins forms with volatile or labile substances stabilize the substances. For example hydrophobic molecules can be assembled in the cavity of Cyclodextrins via noncovalent interactions. In many cases the mechanism of controlled degradation of such complexes is based on pH change of water solutions, leading to the loss of hydrogen or ionic bonds between the host.

Alpha-, beta-, and gamma-cyclodextrin are all generally recognized as safe and non-toxic. Various cyclodextrins are disclosed in U.S. Patent No. 9,493,582 to Antle et al., issued November 15, 2016, and U.S. Patent No. 9,492,552 to Pasloske et al., issued November 15, 2016, which are incorporated herein by reference.

Cyclodextrin are known to be used in many hygiene products such as diapers, products to trap odor causing compounds, thereby reducing the odor.

Cyclodextrin are known to bind fragrance compounds and be capable of releasing fragrances as when heated as in the case of many known dryer sheets in which heat from a clothes dryer releases the fragrance compound into the clothing.

Cyclodextrins are known to be used to produce alcohol powder by encapsulating ethanol. The powder produces an alcoholic beverage when mixed with water. An alcohol powder is disclosed in U.S. Patent No. 3,795,747 to Mitchell et al., issued March 5, 1974, which is incorporated herein by reference.

Wetted sanitary wipes are known as sheets provided impregnated or soaked in a suitable cleaning fluid and ready for use as in a sealed container with the wipe sheet ready to be used once the wipe sheet is removed from the container. Such wipe sheets include for example wipes suitable for cleaning as for example as baby wipes for cleaning infants when changing diapers. Such known wet wipes include those taught by US Patent 5,540,332 to Kopacz, issued July 30, 1996. It is known to package a plurality of such wipes sheets in a single container with a removable sealing lid or closure as taught, for example, in US Patent 3,921,802 to Thompson, issued November 25, 1975; US Patent 4,156,493 to Julius, issued May 29, 1979 and US Patent 5,785,179 to Buczwinski et al, issued July 28, 1998, the disclosures of which are incorporated herein by reference.

The applicant has appreciated that particularly when the cleaning fluid includes volatile compounds that such packages suffer the disadvantage that the fluid will tend to diffuse from the package even when unopened leading to a limited shelf life of the product. Loss of the fluid and drying out of the wipe sheets rendering them unsuitable for use is particularly a disadvantage after initial opening of the package as with the passage of time the fluid will diffuse from the container at the least via the lid or removable closure even when reapplied. The present applicant has appreciated the disadvantage that with the passage of time known wipes become dry and fail to serve as a useful wet wipe.

The applicants have appreciated that previously known wipe sheet devices suffer the disadvantage that they do not provide a wetted wipe with volatile alcohol compounds that can be stored for extensive time yet be activated by the mere application of water and include advantageous volatile compounds including ethanol and other alcohols.

### Summary of the Invention

To at least partially overcome some of these disadvantages of the previously known devices, the present invention provides a wipe that carries or is impregnated with cyclodextrin compounds that retain one or more cleaning and/or disinfecting compounds, such as ethanol, which are released on the wetting of the wipe with water. Preferably, the wipe, in accordance with the present invention, is also useful for drying wetted surfaces such as wet user's hands and countertop and table surfaces serving to, at least partially, dry the surfaces by removing the fluid containing water from the surfaces and releasing the cleaning and/or disinfecting compounds such as ethanol to clean and disinfect the surface.

A preferred method of use of the wipe in accordance with the present invention is to wet the wipe with a fluid containing water to release the cleaning and/or disinfecting compound while engaging a surface to be cleaned with the wetted wipe with released cleaning and/or disinfecting compounds to engage the surface to be cleaned. Preferably, the wipe is in a sheet-like form as in a wipe sheet.

To at least partially overcome some of these disadvantages, the present invention also provides a method of cleansing a surface comprising wetting a surface with a fluid containing water and engaging the wetted surface with a wipe that carries or is impregnated with cyclodextrin compounds that contain one or more cleaning and/or disinfecting compounds which are released on wetting the wipe with water from the fluid. Preferably, the step of wetting the surface comprises washing and/or disinfecting the surface with the fluid containing the water as a first cleaning step and, as second cleaning step, the wipe is wetted by the water from the fluid releasing the cleaning and/or disinfecting compounds in the wipe which released compounds are engaged with the surface to be cleaned by movement of the wipe in engagement with the surface. Preferably, the movement and engagement of the wipe with the surface provides for mechanical cleaning action. Preferably, the engagement and movement of the wipe sheet with the surface also absorbs water from the fluid serving at least partially to dry the surface. Insofar as the surface to be cleaned after engagement by the wetted wipe has fluid left on the surface to be cleaned, advantageously, such remaining fluid is volatile, such as comprising volatile alcohols, which will evaporate readily preferably avoiding the need for further drying by liquid removal.

To at least partially overcome some of these disadvantages, the present invention provides a container containing one or more wipes, preferably as sheets that carry or are impregnated with cyclodextrin compounds that retain cleaning and/or disinfecting compounds, such as ethanol, which are released on the wetting of the wipe with water. A wipe may be removed from the container and wet with water, releasing the volatile component into the water to form a solution of the water and the component. The wipe containing container may be provided with a removable and replaceable closure that forms a seal with an opening to the container. When the wipes are desired to be used, the container containing the wipes is filled with water, and the closure applied to the container to seal it. The water in the container releases the volatile component into the water to form a solution of the water and the component impregnating the wipes, rendering the wipes in the container ready for use.

The wipes may be wet with a fluid containing water. Such fluid includes water alone and fluids containing water and other liquids such as water and alcohol solutions, fluids containing water and soap, fluids prepared by mixing water and soap, and fluids containing water, possibly other liquids and solid particles as, for example, a slurry or suspension such as of the fluid and pumice particles or a colloidal suspension of soap particles.

The wipes may be provided and stored open to the atmosphere in an effectively dry state, however, it is preferred that the wipes be stored sealed from the atmosphere and environment about the wipes, and to avoid any loss of the cleaning and/or disinfecting compounds from the wipes to the atmosphere and environment.

The wipes are preferably stored in packaging that provides for resistance to transfer of the volatile component from the wipe to the atmosphere and for resistance of transfer of water from the atmosphere into the package. For example, one or more wipes may be sealed within a simple package or envelope of suitably impermeable sheeting, preferably hermetically. The packaging may be an envelope, for example, of a plastic sheet that is to be cut or torn for one time opening, or may have a resealable opening. A wipe may be removed from the packaging and wetted with water and then promptly used. The wipes are preferably disposable and intended to be disposed after use.

A plurality of wipes may be provided sealed in a container having a removable sealing closure such as a cap or lid. When the wipes are desired to be used, the container is opened by removing the lid, a suitable amount of water is added to the container, the lid replaced and the container shaken or left to sit such that the water will activate the wipes by releasing and dissolving the compounds, and the resultant water solution come to be uniformly distributed throughout the wipes. The wipes are then ready for use as by being drawn one by one from the container with the closure lid being replaced after each wipe is removed.

The wipes may be provided sealed in a packaging and then provided in combination with a container having a removable sealing closure such as a cap or lid. The wipes may be removed from the packaging and placed into the container with water to activate the wipes. The packaging may be sized to be provided within the container for shipment before opening and use. A plurality of packages may be provided to fit for shipment within one container, with the packages to be removed from the container and, preferably, one package being opened and its wipes placed in the container for activation by adding water and use and the other packages stored for later use.

The container may provide an interior cavity of a volume adequate to store one or a plurality of the wipes and/or a package or packages of the wipes. The container cavity may provide a volume such that with an allotment of the wipes in the cavity there is an adequate additional volume suitable for an appropriate amount of water. The wipes may, for example, have holes or openings or a porosity adequate to promptly receive a volume added to the cavity. The container in one form may have a cavity whose volume is expandable from an initial volume to contain merely an allotment of the wipes to an enlarged volume with includes an additional volume suitable for the water as, for example, the container having bellows-type or accordion-like walls which are expandable to increase the volume.

In one method of use, a wipe is supplied effectively dry and unwetted, and, when desired to be used, is wetted and shortly after being wetted is used for its purpose. In another method, one or a plurality of wipes are wetted at the same time as, for example, within a sealable container, and the one or plurality of wetted wipes are stored in the container ready for use, with individual wipes being available by removing and reapplying the sealing closure to the container. After wetting, the wipes are kept in the container with the sealing closure applied, and the wipes will remain wetted in the sealed container for a reasonable period of time for use.

The cleaning and/or disinfecting compounds preferably are selected from known such compounds including ethanol and isopropanol, with a particularly preferred compound being ethanol. It is known by persons skilled in the art to select suitable cyclodextrins that will encapsulate various different cleaning and/or disinfecting compounds and to select various different cleaning and/or disinfecting compounds that can be encapsulated by suitable cyclodextrins.

Cyclodextrins are preferably used which encapsulate the cleaning and/or disinfecting compounds, preferably ethanol, in a dry form and which, on the addition of water, produces a solution, for example, of an ethanol and water solution. Such cyclodextrins can contain varying amounts of retained compounds. For example, up to 60% by weight ethanol that is a ratio by weight of ethanol to cyclodextrins of 6:4. Cyclodextrins which encapsulate ethanol are capable of producing, for example, when wetted with suitable amounts of water, alcohol water solutions of varying ethanol concentration, for example, in the range of 10% to 70% alcohol by volume, including at least 10%, 20%, 30%, 40%, 50%, 60% and 70%, depending on the relative amounts of water added.

While ethanol concentrations in water in excess of 60% and, preferably, not greater than 85%, are advantageous for killing many pathogens on contact, lower concentration ethanol solutions are excellent as cleaners and disinfectants in hand cleaning operations as in the manner of using a hand soap. Preferred wipes in accordance with the present invention are for use in cleaning hands and skin surfaces and have alcohol water solutions in the range of 10% to 40% alcohol by volume.

The wipe preferably includes a substrate which will become wetted as by absorbing the water when added, permit the water and the cyclodextrins carrying the ethanol to come into contact to release the ethanol into the water forming a solution of water and alcohol which solution is retained by the substrate, such that when the substrate is brought into contact with a surface to be cleaned, the solution is brought into contact with and/or discharged onto the surface. There is no particular limit to the nature of the substrate and it may, for example, preferably comprise a sheet, paper towel-like member, a woven fabric, or a mat of bonded fibers, a porous sponge-like member and the like. Preferably, the substrate may be in the form of a sheet, although this is not necessary.

The wipes preferably when brought into contact with a fluid containing water absorb the water into the wipe in the sense that the water is taken up by the wipe. The absorption of the water by the substrate of the wipe assists in, for example, transferring water engaged with the exterior surface of the wipe into the wipe for release of the cleaning and/or disinfecting compounds to the exterior surfaces of the wipe for engagement with a surface to be cleaned and disinfected. In the situation where the surface to be cleaned is wet with water before engagement by the wipe and preferably with the wipe to be wet merely by the water on the surface, the absorption of the water by the wipe assists in providing a drying action to the surface to be cleaned to remove the water from the surface to be cleaned, preferably by rubbing the wipe over the surface to be cleaned. The absorbency of the wipe, notably as established by the selection of the properties of the substrate, is to be selected by a person skilled in the art as by experimentation so as, on one hand, to assist in placing water in engagement with the wipe to release the cleaning and/or disinfecting compounds to permit the released cleaning and/or disinfecting compounds to become available to the surface of the wipe to engage the surface to be cleaned, as by compression of the wipe into the surface to be cleaned and, if also desired, to dry the surface to be cleaned by absorption of water from the surface to be cleaned. The wipes also preferably have an exterior surface suitable for rubbing the surface to be cleaned to provide for mechanical cleaning. The wipes preferably are made to withstand forces arising in normal forces occurring in rubbing on the surface to be cleaned without destruction of the wipes. Preferably, the wipes have a flexibility which assists in the wipes in being rubbed on the surface to be cleaned engaging the surface and, for example, capable of deforming to engage the varying contours of the surface to be cleaned such as the varying surfaces of a person's hand.

The wipes may merely have one or more cleaning and/or disinfecting compounds. However, the wipes may include various other supplemental compounds such as those that function as fragrances, hand lotions, lubricants, skin moisteners and the like depending on the desired purpose of the wipes. These other compounds may be compounds which are also encapsulated by cyclodextrins as may be advantageous if, for example, the other compounds are volatile. The other compounds may be non-volatile compounds which remain carried by the substrate in dry form without any substantial loss over time and come to be carried by or dissolved into the water solution when the water is added.

Preferably, one or more cleaning and/or disinfecting compounds and the supplemental compounds may all be carried by or incorporated into the dry wipes. This is, however, not necessary. For example, in the case that one or more wipes are to be placed inside a container and water added to the container, the one or more cleaning and/or disinfecting compounds and the supplemental compounds may be in a substantially non-volatile form within the container as, for example, in a dry powder form. For example, a dry powder of some or all of the cleaning and/or disinfecting compounds and the supplemental compounds may be provided in the container suitable for an allotment of one or more wipes to also be placed into the container with a desired amount of water. The dry powder can include a powder of cyclodextrins carrying the cleaning and/or disinfecting compounds and the supplemental compounds. Once water is added to the container, preferably, the water is permitted to wet and dissolve and mix all of the cleaning and/or disinfecting compounds and the supplemental compounds in the wipes and in any powder for uniform distribution throughout the wipes, as may be accommodated by attaching a sealable closure to the container, agitating as by shaking the container, and waiting at least some minimal time before use of the wipes.

The wipes may be used for a variety of purposes with the particular nature of the wipe and the compounds encapsulated by cyclodextrins selected having regard to the intended usage and purpose. For example, for use in hand cleaning, the wipe may be configured to produce on the addition of water an ethanol water solution having in the range of 20% to 40% ethanol in water. As another example, where the wipe is to be used to disinfect a wound, the wipe may be configured to produce on the addition of water an ethanol water solution having in the range of 50% to 70% ethanol in water. The wipe may have other disinfecting compounds than ethanol and such other disinfecting compounds are to be selected having regard to the intended purpose of the wipe.

Accordingly, in a first aspect the present invention resides in a wipe comprising: a substrate; and a cleaning and/or disinfecting compound retained by one or more cyclodextrin compounds.

In a second aspect, the invention resides in a wipe, which optionally incorporates one or more features of the first aspect, wherein the one or more cyclodextrin compounds release the cleaning and/or disinfecting compound upon wetting the wipe with water.

In a third aspect, the invention resides in a wipe, which optionally incorporates one or more features of any one or more of the first and second aspects, wherein the cleaning and/or disinfecting compound comprises an alcohol.

In a fourth aspect, the invention resides in a wipe, which optionally incorporates one or more features of any one or more of the first to third aspects, wherein the alcohol comprises ethanol.

In a fifth aspect, the invention resides in a wipe, which optionally incorporates one or more features of any one or more of the first to fourth aspects, wherein the substrate carries or is impregnated with the cleaning and/or disinfecting compound retained by the one or more cyclodextrin compounds.

In a sixth aspect, the invention resides in a wipe, which optionally incorporates one or more features of any one or more of the first to fifth aspects, wherein the cleaning and/or disinfecting compound retained by the one or more cyclodextrin compounds is in a dry powdered form.

In a seventh aspect, the invention resides in a wipe, which optionally incorporates one or more features of any one or more of the first to sixth aspects, wherein the wipe is stored in a dry state.

In an eighth aspect, the invention resides in a wipe, which optionally incorporates one or more features of any one or more of the first to seventh aspects, wherein the substrate is capable of absorbing a fluid.

In a ninth aspect, the invention resides in a wipe, which optionally incorporates one or more features of any one or more of the first to eighth aspects, wherein the substrate has at least one of: holes, pores, and openings for absorbing the fluid.

In a tenth aspect, the invention resides in a wipe, which optionally incorporates one or more features of any one or more of the first to ninth aspects, wherein the substrate comprises at least one of: a sheet, a paper towel-like member, a woven fabric, a mat of bonded fibers, and a porous sponge-like member.

In an eleventh aspect, the invention resides in a method of using a wipe, which optionally incorporates one or more features of any one or more of the first to tenth aspects, the method comprising: wetting the wipe with one or more fluids comprising water to release the cleaning and/or disinfecting compound from the one or more cyclodextrin compounds; and applying the wipe to a surface to clean and/or disinfect the surface.

In a twelfth aspect, the invention resides in a method of using a wipe, which optionally incorporates one or more features of any one or more of the first to eleventh aspects, wherein the one or more fluids comprising water includes a surface fluid; wherein, before the wipe is applied to the surface, the surface fluid is present on the surface; and wherein wetting the wipe comprises applying the wipe to the surface fluid that is present on the surface.

In a thirteenth aspect, the invention resides in a method of using a wipe, which optionally incorporates one or more features of any one or more of the first to twelfth aspects, further comprising applying the surface fluid to the surface.

In a fourteenth aspect, the invention resides in a method of using a wipe, which optionally incorporates one or more features of any one or more of the first to thirteenth aspects, further comprising drying the surface with the wipe.

In a fifteenth aspect, the invention resides in a method of using a wipe, which optionally incorporates one or more features of any one or more of the first to fourteenth aspects, wherein drying the surface comprises absorbing the surface fluid into the substrate.

In a sixteenth aspect, the invention resides in a method of using a wipe, which optionally incorporates one or more features of any one or more of the first to fifteenth aspects, wherein applying the wipe to the surface comprises engaging the cleaning and/or disinfecting compound with the surface by movement of the wipe in engagement with the surface.

In a seventeenth aspect, the invention resides in a method of using a wipe, which optionally incorporates one or more features of any one or more of the first to sixteenth aspects, wherein the cleaning and/or disinfecting compound is volatile, so that, after the wipe is applied to the surface, any of the cleaning and/or disinfecting compound that remains on the surface dries by evaporation.

In an eighteenth aspect, the invention resides in a method of using a wipe, which optionally incorporates one or more features of any one or more of the first to seventeenth aspects, further comprising storing the wipe in a container; wherein the one or more fluids comprising water includes a container fluid; and wherein wetting the wipe comprises adding the container fluid to the container.

In a nineteenth aspect, the invention resides in a method of using a wipe, which optionally incorporates one or more features of any one or more of the first to eighteenth aspects, wherein storing the wipe in the container comprises storing the wipe in the container for a first period of time, before wetting the wipe with the one or more fluids comprising water; the method further comprising storing the wipe in the container for a second period of time, after the container fluid is added to the container.

In a twentieth aspect, the invention resides in a method of using a wipe, which optionally incorporates one or more features of any one or more of the first to nineteenth aspects, wherein wetting the wipe comprises placing the wipe and at least one of the one or more fluids comprising water into a container.

In a twenty first aspect, the invention resides in a method of using a wipe, which optionally incorporates one or more features of any one or more of the first to twentieth aspects, further comprising storing the wipe in the container with the at least one of the one or more fluids comprising water.

In a twenty second aspect, the invention resides in a method of using a wipe, which optionally incorporates one or more features of any one or more of the first to twenty first aspects, wherein the one or more fluids comprising water includes a soaking fluid; and wherein wetting the wipe comprises soaking the wipe in a volume of the soaking fluid to produce, upon release of the cleaning and/or disinfecting compound from the one or more cyclodextrin compounds, a solution containing water and the cleaning and/or disinfecting compound.

In a twenty third aspect, the invention resides in a method of using a wipe, which optionally incorporates one or more features of any one or more of the first to twenty second aspects, wherein the solution containing water and the cleaning and/or disinfecting compound contains at least 10% of the cleaning and/or disinfecting compound by volume.

In a twenty fourth aspect, the invention resides in a method of using a wipe, which optionally incorporates one or more features of any one or more of the first to twenty third aspects, wherein the solution containing water and the cleaning and/or disinfecting compound contains 10% to 70% of the cleaning and/or disinfecting compound by volume.

In a twenty fifth aspect, the invention resides in a method of using a wipe, which optionally incorporates one or more features of any one or more of the first to twenty fourth aspects, wherein the solution containing water and the cleaning and/or disinfecting compound contains 10% to 40% of the cleaning and/or disinfecting compound by volume.

In a twenty sixth aspect, the invention resides in a method of using a wipe, which optionally incorporates one or more features of any one or more of the first to twenty fifth aspects, wherein the solution containing water and the cleaning and/or disinfecting compound contains no more than 85% of the cleaning and/or disinfecting compound by volume.

In a twenty seventh aspect, the invention resides in a container, which optionally incorporates one or more features of any one or more of the first to twenty sixth aspects, the container containing: one or more wipes; and a cleaning and/or disinfecting compound retained by one or more cyclodextrin compounds.

In a twenty eighth aspect, the invention resides in a container, which optionally incorporates one or more features of any one or more of the first to twenty seventh aspects, wherein the cleaning and/or disinfecting compound retained by the one or more cyclodextrin compounds is contained in the container in a dry state.

In a twenty ninth aspect, the invention resides in a container, which optionally incorporates one or more features of any one or more of the first to twenty eighth aspects, wherein the cleaning and/or disinfecting compound retained by the one or more cyclodextrin compounds is contained in the container in a dry powdered form.

In a thirtieth aspect, the invention resides in a container, which optionally incorporates one or more features of any one or more of the first to twenty ninth aspects, wherein the one or more wipes are contained in the container in a dry state.

In a thirty first aspect, the invention resides in a container, which optionally incorporates one or more features of any one or more of the first to thirtieth aspects, wherein the one or more cyclodextrin compounds release the cleaning and/or disinfecting compound upon being dissolved in a fluid comprising water.

In a thirty second aspect, the invention resides in a container, which optionally incorporates one or more features of any one or more of the first to thirty first aspects, wherein the cleaning and/or disinfecting compound comprises an alcohol.

In a thirty third aspect, the invention resides in a container, which optionally incorporates one or more features of any one or more of the first to thirty second aspects, wherein the alcohol comprises ethanol.

In a thirty fourth aspect, the invention resides in a container, which optionally incorporates one or more features of any one or more of the first to thirty third aspects, wherein the one or more wipes comprise the wipe in accordance with one or more of the first to tenth aspects.

In a thirty fifth aspect, the invention resides in a method of using a wipe, which optionally incorporates one or more features of any one or more of the first to thirty fourth aspects, the method comprising: storing the wipe in a container; storing a cleaning and/or disinfecting compound retained by one or more cyclodextrin compounds in the container; placing a fluid comprising water into the container, the one or more cyclodextrin compounds releasing the cleaning and/or disinfecting compound upon being dissolved in the fluid comprising water to produce a solution containing water and the cleaning and/or disinfecting compound; removing the wipe from the container, the wipe carrying at least some of the solution containing water and the cleaning and/or disinfecting compound; and applying the wipe to a surface to clean and/or disinfect the surface.

In a thirty sixth aspect, the invention resides in a method of using a wipe, which optionally incorporates one or more features of any one or more of the first to thirty fifth aspects, the method comprising: providing a container containing the wipe and a cleaning and/or disinfecting compound retained by one or more cyclodextrin compounds; placing a fluid comprising water into the container, the one or more cyclodextrin compounds releasing the cleaning and/or disinfecting compound upon being dissolved in the fluid comprising water to produce a solution containing water and the cleaning and/or disinfecting compound; removing the wipe from the container, the wipe carrying at least some of the solution containing water and the cleaning and/or disinfecting compound; and applying the wipe to a surface to clean and/or disinfect the surface.

In a thirty seventh aspect, the invention resides in a method of using a wipe, which optionally incorporates one or more features of any one or more of the first to thirty sixth aspects, the method comprising: storing the wipe in a container; storing a cleaning and/or disinfecting compound retained by one or more cyclodextrin compounds in the container; storing a fluid comprising water in the container, the fluid comprising water being contained in a separate compartment from the cleaning and/or disinfecting compound retained by the one or more cyclodextrin compounds; mixing the fluid comprising water with the cleaning and/or disinfecting compound retained by the one or more cyclodextrin compounds, the one or more cyclodextrin compounds releasing the cleaning and/or disinfecting compound upon being dissolved in the fluid comprising water to produce a solution containing water and the cleaning and/or disinfecting compound; removing the wipe from the container, the wipe carrying at least some of the solution containing water and the cleaning and/or disinfecting compound; and applying the wipe to a surface to clean and/or disinfect the surface.

In a thirty eighth aspect, the invention resides in a method of using a wipe, which optionally incorporates one or more features of any one or more of the first to thirty seventh aspects, wherein mixing the fluid comprising water with the cleaning and/or disinfecting compound retained by the one or more cyclodextrin compounds comprises breaking a seal separating the fluid comprising water from the cleaning and/or disinfecting compound retained by the one or more cyclodextrin compounds.

In a thirty ninth aspect, the invention resides in a method of using a wipe, which optionally incorporates one or more features of any one or more of the first to thirty eighth aspects, wherein the container comprises the container in accordance with any one or more of the twenty seventh to thirty fourth aspects.

In a fortieth aspect, the invention resides in a method of using a wipe, which optionally incorporates one or more features of any one or more of the first to third aspects, the method comprising: providing a cleaning and/or disinfecting compound retained by one or more cyclodextrin compounds; dissolving the cleaning and/or disinfecting compound retained by the one or more cyclodextrin compounds in a fluid comprising water, the one or more cyclodextrin compounds releasing the cleaning and/or disinfecting compound upon being dissolved in the fluid comprising water to produce a solution containing water and the cleaning and/or disinfecting compound; carrying, in or on the wipe, at least some of the solution containing water and the cleaning and/or disinfecting compound; and applying the wipe to a surface to clean and/or disinfect the surface.

In a forty first aspect, the invention resides in a method of using a wipe, which optionally incorporates one or more features of any one or more of the first to fortieth aspects, wherein providing the cleaning and/or disinfecting compound retained by the one or more cyclodextrin compounds comprises providing the cleaning and/or disinfecting compound retained by the one or more cyclodextrin compounds in a dry state.

In a forty second aspect, the invention resides in a method of using a wipe, which optionally incorporates one or more features of any one or more of the first to forty first aspects, wherein providing the cleaning and/or disinfecting compound retained by the one or more cyclodextrin compounds comprises providing the cleaning and/or disinfecting compound retained by the one or more cyclodextrin compounds in a dry powdered form.

In a forty third aspect, the invention resides in a method of using a wipe, which optionally incorporates one or more features of any one or more of the first to forty second aspects, wherein the cleaning and/or disinfecting compound comprises an alcohol.

In a forty fourth aspect, the invention resides in a method of using a wipe, which optionally incorporates one or more features of any one or more of the first to forty third aspects, wherein the alcohol comprises ethanol.

In a forty fifth aspect, the invention resides in a method of using a wipe, which optionally incorporates one or more features of any one or more of the first to forty fourth aspects, wherein the wipe comprises the wipe in accordance with any one or more of the first to tenth aspects.

In a forty sixth aspect, the invention resides in a wipe, which optionally incorporates one or more features of any one or more of the first to forty fifth aspects, the wipe comprising: a substrate; and an active ingredient retained by one or more cyclodextrin compounds.

In a forty seventh aspect, the invention resides in a wipe, which optionally incorporates one or more features of any one or more of the first to forty sixth aspects, wherein the active ingredient comprises at least one of: a cleaning compound, a disinfecting compound, and a cleaning and disinfecting compound.

In a forty eighth aspect, the invention resides in a wipe, which optionally incorporates one or more features of any one or more of the first to forty seventh aspects, wherein the one or more cyclodextrin compounds release the active ingredient upon wetting the wipe with water.

In a forty ninth aspect, the invention resides in a wipe, which optionally incorporates one or more features of any one or more of the first to forty eighth aspects, wherein the active ingredient comprises an alcohol.

In a fiftieth aspect, the invention resides in a wipe, which optionally incorporates one or more features of any one or more of the first to forty ninth aspects, the wipe comprising: a substrate; and an alcohol powder.

In a fifty first aspect, the invention resides in a wipe, which optionally incorporates one or more features of any one or more of the first to fiftieth aspects, wherein the alcohol powder comprises ethanol in a dry powdered form.

In a fifty second aspect, the invention resides in a wipe, which optionally incorporates one or more features of any one or more of the first to fifty first aspects, wherein the substrate carries or is impregnated with the alcohol powder.

In a fifty third aspect, the invention resides in a wipe, which optionally incorporates one or more features of any one or more of the first to fifty second aspects, wherein the wipe is stored in a dry state.

In a fifty fourth aspect, the invention resides in a method of using a wipe, which optionally incorporates one or more features of any one or more of the first to fifty third aspects, the method comprising: wetting the wipe with one or more fluids comprising water to dissolve the alcohol powder and thereby produce a solution comprising alcohol; and applying the wipe to a surface to deposit at least some of the solution comprising alcohol onto the surface.

In a fifty fifth aspect, the invention resides in a method of using a wipe, which optionally incorporates one or more features of any one or more of the first to fifty fourth aspects, wherein the solution comprising alcohol contains at least 10% of the alcohol by volume.

In a fifty sixth aspect, the invention resides in a method of using a wipe, which optionally incorporates one or more features of any one or more of the first to fifty fifth aspects, wherein the solution comprising alcohol contains 10% to 70% of the alcohol by volume.

In a fifty seventh aspect, the invention resides in a method of using a wipe, which optionally incorporates one or more features of any one or more of the first to fifty sixth aspects, wherein the solution comprising alcohol contains 10% to 40% of the alcohol by volume.

In a fifty eighth aspect, the invention resides in a method of using a wipe, which optionally incorporates one or more features of any one or more of the first to fifty seventh aspects, wherein the solution comprising alcohol contains no more than 85% of the alcohol by volume.

In a fifty ninth aspect, the invention resides in a wipe, which optionally incorporates one or more features of any one or more of the first to fifty eighth aspects, wherein the wipe is at least one of: a hand cleaning wipe; a human skin cleaning wipe; an infant human skin cleaning wipe; a counter top wipe; a table top wipe; a floor wipe; a wall wipe; a furniture wipe; an instrument wipe; and an apparatus wipe.

In a sixtieth aspect, the invention resides in a wipe, which optionally incorporates one or more features of any one or more of the first to fifty ninth aspects, the wipe comprising: a substrate; and ethanol retained by one or more cyclodextrin compounds; wherein the one or more cyclodextrin compounds release the ethanol upon wetting the wipe with water; and wherein the substrate carries or is impregnated with the ethanol retained by the one or more cyclodextrin compounds.

In a sixty first aspect, the invention resides in a wipe, which optionally incorporates one or more features of any one or more of the first to sixtieth aspects, wherein the ethanol retained by the one or more cyclodextrin compounds is in a dry powdered form.

In a sixty second aspect, the invention resides in a wipe, which optionally incorporates one or more features of any one or more of the first to sixty first aspects, wherein the wipe is stored in a dry state.

In a sixty third aspect, the invention resides in a wipe, which optionally incorporates one or more features of any one or more of the first to sixty second aspects, wherein the substrate is capable of absorbing a fluid.

In a sixty fourth aspect, the invention resides in a wipe, which optionally incorporates one or more features of any one or more of the first to sixty third aspects, wherein the substrate has at least one of: holes, pores, and openings for absorbing the fluid.

In a sixty fifth aspect, the invention resides in a wipe, which optionally incorporates one or more features of any one or more of the first to sixty fourth aspects, wherein the substrate comprises at least one of: a sheet, a paper towel-like member, a woven fabric, a mat of bonded fibers, and a porous sponge-like member.

In a sixty sixth aspect, the invention resides in a method of using a wipe, which optionally incorporates one or more features of any one or more of the first to sixty fifth aspects, the method comprising: wetting the wipe with one or more fluids comprising water to release the ethanol from the one or more cyclodextrin compounds; and applying the wipe to a surface to clean and/or disinfect the surface.

In a sixty seventh aspect, the invention resides in a method of using a wipe, which optionally incorporates one or more features of any one or more of the first to sixty sixth aspects, wherein the one or more fluids comprising water includes a surface fluid; wherein, before the wipe is applied to the surface, the surface fluid is present on the surface; and wherein wetting the wipe comprises applying the wipe to the surface fluid that is present on the surface.

In a sixty eighth aspect, the invention resides in a method of using a wipe, which optionally incorporates one or more features of any one or more of the first to sixty seventh aspects, further comprising applying the surface fluid to the surface.

In a sixty ninth aspect, the invention resides in a method of using a wipe, which optionally incorporates one or more features of any one or more of the first to sixty eighth aspects, further comprising drying the surface with the wipe; wherein drying the surface comprises absorbing the surface fluid into the substrate.

In a seventieth aspect, the invention resides in a method of using a wipe, which optionally incorporates one or more features of any one or more of the first to sixty ninth aspects, wherein applying the wipe to the surface comprises engaging the ethanol with the surface by movement of the wipe in engagement with the surface.

In a seventy first aspect, the invention resides in a method of using a wipe, which optionally incorporates one or more features of any one or more of the first to seventieth aspects, further comprising storing the wipe in a container; wherein the one or more fluids comprising water includes a container fluid; and wherein wetting the wipe comprises adding the container fluid to the container.

In a seventy second aspect, the invention resides in a method of using a wipe, which optionally incorporates one or more features of any one or more of the first to seventy first aspects, wherein storing the wipe in the container comprises storing the wipe in the container for a first period of time, before wetting the wipe with the one or more fluids comprising water; the method further comprising storing the wipe in the container for a second period of time, after the container fluid is added to the container.

In a seventy third aspect, the invention resides in a method of using a wipe, which optionally incorporates one or more features of any one or more of the first to seventy second aspects, wherein the one or more fluids comprising water includes a soaking fluid; and wherein wetting the wipe comprises soaking the wipe in a volume of the soaking fluid to produce, upon release of the ethanol from the one or more cyclodextrin compounds, a solution containing water and ethanol.

In a seventy fourth aspect, the invention resides in a method of using a wipe, which optionally incorporates one or more features of any one or more of the first to seventy third aspects, wherein the solution containing water and ethanol contains at least one of: at least 10% ethanol by volume; 10% to 70% ethanol by volume; 10% to 40% ethanol by volume; and no more than 85% ethanol by volume.

In a seventy fifth aspect, the invention resides in a wipe, which optionally incorporates one or more features of any one or more of the first to seventy fourth aspects, the wipe comprising: a substrate; and ethanol retained by one or more cyclodextrin compounds; wherein the one or more cyclodextrin compounds release the ethanol upon wetting the wipe with water; and wherein the substrate carries or is impregnated with the ethanol retained by the one or more cyclodextrin compounds.

In a seventy sixth aspect, the invention resides in a wipe, which optionally incorporates one or more features of any one or more of the first to seventy fifth aspects, wherein the ethanol retained by the one or more cyclodextrin compounds is in a dry powdered form.

In a seventy seventh aspect, the invention resides in a wipe, which optionally incorporates one or more features of any one or more of the first to seventy sixth aspects, wherein the wipe is stored in a dry state.

In a seventy eighth aspect, the invention resides in a wipe, which optionally incorporates one or more features of any one or more of the first to seventy seventh aspects, wherein the substrate is capable of absorbing a fluid.

In a seventy ninth aspect, the invention resides in a wipe, which optionally incorporates one or more features of any one or more of the first to seventy eighth aspects, wherein the substrate has at least one of: holes, pores, and openings for absorbing the fluid.

In an eightieth aspect, the invention resides in a wipe, which optionally incorporates one or more features of any one or more of the first to seventy ninth aspects, wherein the substrate comprises at least one of: a sheet, a paper towel-like member, a woven fabric, a mat of bonded fibers, and a porous sponge-like member.

In an eighty first aspect, the invention resides in a method of using a wipe, which optionally incorporates one or more features of any one or more of the first to eightieth aspects, the method comprising: wetting the wipe with one or more fluids comprising water to release the ethanol from the one or more cyclodextrin compounds; and applying the wipe to a surface to clean and/or disinfect the surface.

In an eighty second aspect, the invention resides in a method of using a wipe, which optionally incorporates one or more features of any one or more of the first to eighty first aspects, wherein the one or more fluids comprising water includes a surface fluid; wherein, before the wipe is applied to the surface, the surface fluid is present on the surface; and wherein wetting the wipe comprises applying the wipe to the surface fluid that is present on the surface.

In an eighty third aspect, the invention resides in a method of using a wipe, which optionally incorporates one or more features of any one or more of the first to eighty second aspects, further comprising applying the surface fluid to the surface.

In an eighty fourth aspect, the invention resides in a method of using a wipe, which optionally incorporates one or more features of any one or more of the first to eighty third aspects, further comprising drying the surface with the wipe; wherein drying the surface comprises absorbing the surface fluid into the substrate.

In an eighty fifth aspect, the invention resides in a method of using a wipe, which optionally incorporates one or more features of any one or more of the first to eighty fourth aspects, wherein applying the wipe to the surface comprises engaging the ethanol with the surface by movement of the wipe in engagement with the surface.

In an eighty sixth aspect, the invention resides in a method of using a wipe, which optionally incorporates one or more features of any one or more of the first to eighty fifth aspects, further comprising storing the wipe in a container; wherein the one or more fluids comprising water includes a container fluid; and wherein wetting the wipe comprises adding the container fluid to the container.

In an eighty seventh aspect, the invention resides in a method of using a wipe, which optionally incorporates one or more features of any one or more of the first to eighty sixth aspects, wherein storing the wipe in the container comprises storing the wipe in the container for a first period of time, before wetting the wipe with the one or more fluids comprising water; the method further comprising storing the wipe in the container for a second period of time, after the container fluid is added to the container.

In an eighty eighth aspect, the invention resides in a method of using a wipe, which optionally incorporates one or more features of any one or more of the first to eighty seventh aspects, wherein the one or more fluids comprising water includes a soaking fluid; and wherein wetting the wipe comprises soaking the wipe in a volume of the soaking fluid to produce, upon release of the ethanol from the one or more cyclodextrin compounds, a solution containing water and ethanol.

In an eighty ninth aspect, the invention resides in a method of using a wipe, which optionally incorporates one or more features of any one or more of the first to eighty eighth aspects, wherein the solution containing water and ethanol contains at least 10% ethanol by volume.

In a ninetieth aspect, the invention resides in a method of using a wipe, which optionally incorporates one or more features of any one or more of the first to eighty ninth aspects, wherein the solution containing water and ethanol contains 10% to 70% ethanol by volume.

In a ninety first aspect, the invention resides in a wipe, which optionally incorporates one or more features of any one or more of the first to ninetieth aspects, wherein the ethanol retained by the one or more cyclodextrin compounds is in a dry powdered form; wherein the wipe is stored in a dry state; wherein the substrate is capable of absorbing a fluid; wherein the substrate has at least one of: holes, pores, and openings for absorbing the fluid; and wherein the substrate comprises at least one of: a sheet, a paper towel-like member, a woven fabric, a mat of bonded fibers, and a porous sponge-like member.

In a ninety second aspect, the invention resides in a method of using a wipe, which optionally incorporates one or more features of any one or more of the first to ninety first aspects, the method comprising: wetting the wipe with one or more fluids comprising water to release the ethanol from the one or more cyclodextrin compounds; and applying the wipe to a surface to clean and/or disinfect the surface.

In a ninety third aspect, the invention resides in a method of using a wipe, which optionally incorporates one or more features of any one or more of the first to ninety second aspects, wherein the one or more fluids comprising water includes a surface fluid; wherein, before the wipe is applied to the surface, the surface fluid is present on the surface; wherein wetting the wipe comprises applying the wipe to the surface fluid that is present on the surface; wherein the method further comprises: applying the surface fluid to the surface; and drying the surface with the wipe; wherein drying the surface comprises absorbing the surface fluid into the substrate; and wherein applying the wipe to the surface comprises engaging the ethanol with the surface by movement of the wipe in engagement with the surface.

In a ninety fourth aspect, the invention resides in a method of using a wipe, which optionally incorporates one or more features of any one or more of the first to ninety third aspects, wherein the one or more fluids comprising water includes a soaking fluid; wherein wetting the wipe comprises soaking the wipe in a volume of the soaking fluid to produce, upon release of the ethanol from the one or more cyclodextrin compounds, a solution containing water and ethanol; and wherein the solution containing water and ethanol contains 10% to 70% ethanol by volume.

In a ninety fifth aspect, the present invention resides in a wipe, a container, and/or a method of using a wipe that combines one or more of the features of one or more of the first to ninety fourth aspects with any one or more of the remaining features of any one or more of the first to ninety fourth aspects

### Brief Description of the Drawings

Further aspects and advantages of the present invention will become apparent from the following description taken together with the accompanying drawings in which:
Figure 1 is a front perspective view of a first embodiment of the invention illustrating a single wipe sheet enclosed within a disposable package;
Figure 2 is a sectional view of the package of Figure 1 taken along section line 2-2';
Figure 3 is a sectional view of the package of Figure 2 illustrating the package in an open position providing access to the wipe for removal;
Figure 4 is a perspective view of a second embodiment of a package containing individual wipes; and
Figure 5 is a sectional view of the package of Figure 4 along section line 5-5'.

### Detailed Description of the Drawings

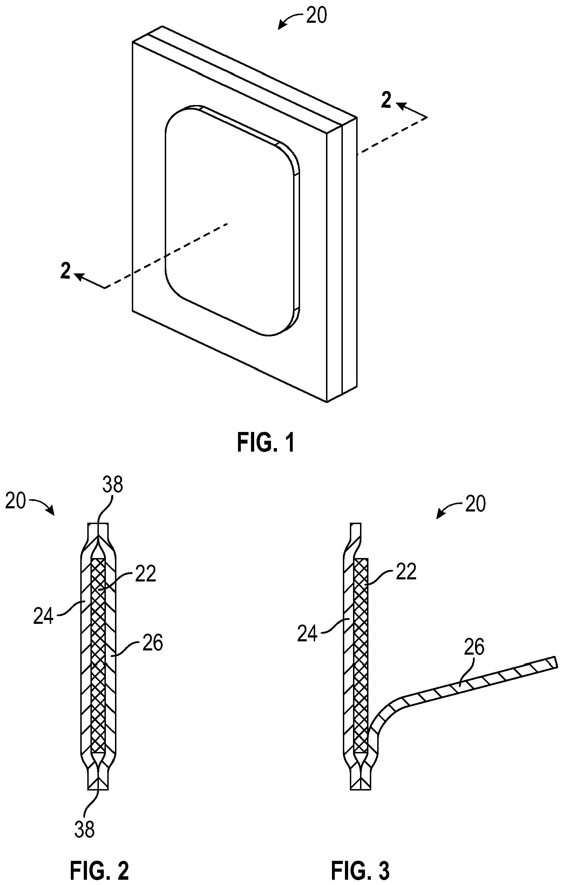

Reference is made to Figures 1 to 3 which show a first embodiment of a package or container 20 containing a single wipe sheet in accordance with the present invention.

As can be seen in Figure 2, the wipe sheet 22 is retained within the package 20 between a pair of sheet members 24 and 26 which are sealed together about their periphery by a peripheral seal 38 so as to sealably retain wipe sheet 22 in a compartment formed within the package 20. For use of the wipe sheet 22, a user tears or breaks the sheets 24 or 26 or the seal 38 therebetween so as to provide access to the wipe sheet 22 as shown in Figure 3 as for removal of the wipe sheet 22. The wipe sheet 22 is preferably substantially dry or free from water and is activated by engagement with water such that the wipe sheet 22 including a substrate is carried or impregnated with cyclodextrin compounds retain a cleaning and/or disinfecting compounds that are released on wetting the wipe sheet 22 with the water. The cleaning and/or disinfecting compound may, for example, comprise an alcohol, such as ethanol.

The wipe 22 is preferably stored in the package 20 in a dry state. The cleaning and/or disinfecting compound retained by one or more cyclodextrin compounds are also preferably stored in a dry state, such as for example a dry powdered form.

To use the wipe 22, the wipe 22 may be removed from the package 20 and wetted with one or more fluids comprising water to release the cleaning and/or disinfecting compound from the one or more cyclodextrin compounds. The wipe 22 can then be applied to a surface, such as a user's hands or a table top, to clean and/or disinfect the surface.

Optionally, the one or more fluids comprising water includes one or more of: a surface fluid, a container fluid, and a soaking fluid. For example, the surface to be cleaned could have a fluid comprising water present on the surface before the wipe 22 is applied to the surface, which fluid can be referred to as a surface fluid. The surface fluid could, for example, be water that a user has applied to their hands, or a spill of a beverage on a table top. By applying the wipe 22 to the surface fluid, the surface fluid wets the wipe 22 and thereby causes the cleaning and/or disinfecting compound to be released from the one or more cyclodextrin compounds. The released cleaning and/or disinfecting compound can then be applied to the surface to clean and/or disinfect the surface, for example by rubbing the wipe 22 over the surface. The wipe 22 also preferably helps to dry the surface by absorbing some or all of the surface fluid.

The wipe 22 could also be wetted by introducing a fluid comprising water to the package 20 before the wipe 22 is removed from the package 20, which fluid can be referred to as a container fluid. For example, after the package 20 is opened as shown in Figure 3 and before the wipe 22 is removed, a fluid such as water could be poured into the package 20, such as by holding the opened package 20 under a water faucet. The pair of sheet members 24 and 26 could then optionally be held together, with the wipe 22 and the fluid contained therebetween, and the package 20 could be shaken to thoroughly disperse the fluid into and around the wipe 22, thereby causing the cleaning and/or disinfecting compound to be released from the one or more cyclodextrin compounds. The wipe 22 could then be removed from the package 20, and applied to a surface to clean and/or disinfect the surface.

The wipe 22 could also be removed from the package 20 in its dry state, and then soaked in a fluid comprising water outside of the package 20 to release the cleaning and/or disinfecting compound from the one or more cyclodextrin compounds. For example, the wipe 22 could, after being removed from the package 20, be held under a water faucet to soak the wipe 22 in water. The wipe 22 could also be placed in a vessel, such as a cup, bowl, or bucket, together with a fluid comprising water, such as a mixture of soap and water, to release the cleaning and/or disinfecting compound from the one or more cyclodextrin compounds.

Any fluid that is used to wet the wipe 22 to release the cleaning and/or disinfecting compound from the one or more cyclodextrin compounds may be referred to as a soaking fluid, regardless of whether said fluid contacts the wipe inside or outside of the package 20. The wipe 22 could be wetted once by one fluid, or more than once by one or more surface fluids, container fluids, and/or soaking fluids, each fluid having the same composition or a different composition.

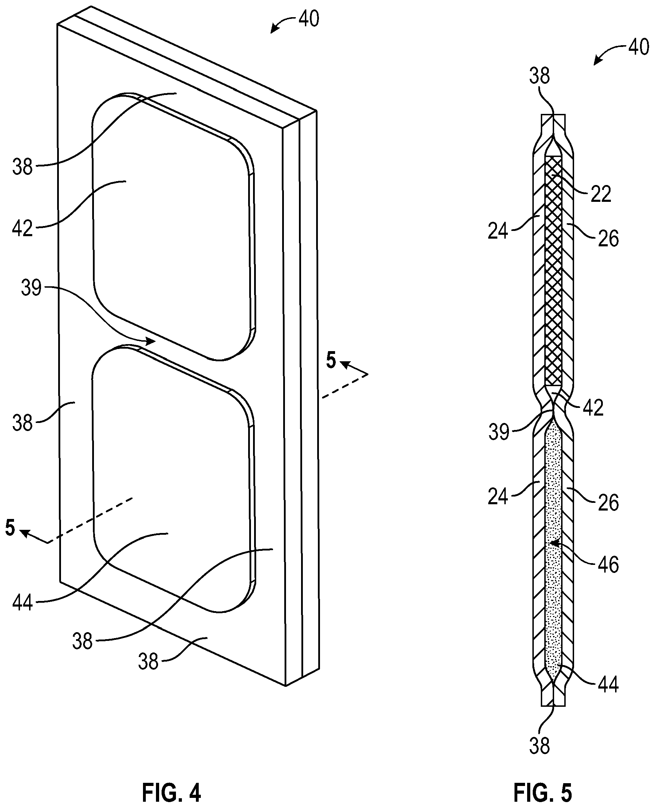

Reference is made to Figures 4 and 5 which illustrate a second embodiment of a package or container 40 for a single wipe sheet 22 which has substantial similarities to the first embodiment of Figures 1 to 3 and similar reference numerals are used to refer to similar elements.

As best seen in Figure 4, two sheets 24 and 26 are sealed about their periphery forming a fluid impermeable seal 38 about the periphery. An intermediate seal 39 extends transversely across the package 40 so as to define within the package 40 an upper compartment 42 and a lower compartment 44. Within the upper compartment 42, the wipe sheet 22 is provided and within the lower compartment 44, a fluid containing water 46, preferably merely water, is contained. The intermediate seal 39 can be broken by manipulation of the package 40 as, for example, by a user pulling the sides of the sheet 24 away from the sheet 26. The intermediate seal 39 is broken under less force than the peripheral seal 38. In use, the intermediate seal 39 is broken, the package 40 is manipulated so that the water from the lower compartment 44 flows to the upper compartment 42 and comes to be engaged with the wipe 22 to wet the wipe 22 and subsequently after manipulation of the package 40 such as by shaking and rubbing to wet the wipe sheet 22, the package 40 is then opened in a manner illustrated in Figure 3 by breaking the peripheral seal 38 and the wet wipe 22 is ready for use. Preferably, if the fluid containing the water in a separate compartment 44 does not include volatile components, the product will be able to be stored for an extended period of time as compared to wipe products in which volatile alcohol is in the wipe in a liquid state.

The substrate for the wipe 22 in accordance with the present invention may comprise a plurality of different materials such as, for example, a lamination of different sheets having selected different properties to provide a composite sheet with desired properties serving the purpose, for example, of carrying or being impregnated with the cyclodextrin compounds to retain the cleaning and/or disinfecting compounds, providing flexibility, resiliency and suitable absorbance by the wipe 22 enhancing the transfer of water from an exterior surface of the wipe 22 to the cyclodextrin compounds and enhancing the transfer of the cleaning and/or disinfecting compounds to the exterior surface of the wipe 22 for engagement with the surface to be cleaned.

The embodiments shown in Figures 1 to 5 therefore provide a wipe 22 comprising: a substrate; and a cleaning and/or disinfecting compound retained by one or more cyclodextrin compounds.

The embodiments shown in Figures 1 to 5 therefore also provide a method of using the aforementioned wipe 22, the method comprising: wetting the wipe 22 with one or more fluids comprising water to release the cleaning and/or disinfecting compound from the one or more cyclodextrin compounds; and applying the wipe 22 to a surface to clean and/or disinfect the surface.

The embodiments shown in Figures 1 to 5 therefore also provide a container 20, 40 containing: one or more wipes; and a cleaning and/or disinfecting compound retained by one or more cyclodextrin compounds.

The embodiments shown in Figures 1 to 5 therefore also provide a method of using a wipe 22, the method comprising: storing the wipe 22 in a container 20, 40; storing a cleaning and/or disinfecting compound retained by one or more cyclodextrin compounds in the container 20, 40; placing a fluid comprising water into the container 20, 40, the one or more cyclodextrin compounds releasing the cleaning and/or disinfecting compound upon being dissolved in the fluid comprising water to produce a solution containing water and the cleaning and/or disinfecting compound; removing the wipe 22 from the container 20, 40, the wipe 22 carrying at least some of the solution containing water and the cleaning and/or disinfecting compound; and applying the wipe 22 to a surface to clean and/or disinfect the surface.

The embodiments shown in Figures 1 to 5 therefore also provide a method of using a wipe 22, the method comprising: providing a container 20, 40 containing the wipe 22 and a cleaning and/or disinfecting compound retained by one or more cyclodextrin compounds; placing a fluid comprising water into the container 20, 40, the one or more cyclodextrin compounds releasing the cleaning and/or disinfecting compound upon being dissolved in the fluid comprising water to produce a solution containing water and the cleaning and/or disinfecting compound; removing the wipe 22 from the container 20, 40, the wipe 22 carrying at least some of the solution containing water and the cleaning and/or disinfecting compound; and applying the wipe 22 to a surface to clean and/or disinfect the surface.

The embodiments shown in Figures 1 to 5 therefore also provide a method of using a wipe 22, the method comprising: providing a cleaning and/or disinfecting compound retained by one or more cyclodextrin compounds; dissolving the cleaning and/or disinfecting compound retained by the one or more cyclodextrin compounds in a fluid comprising water, the one or more cyclodextrin compounds releasing the cleaning and/or disinfecting compound upon being dissolved in the fluid comprising water to produce a solution containing water and the cleaning and/or disinfecting compound; carrying, in or on the wipe 22, at least some of the solution containing water and the cleaning and/or disinfecting compound; and applying the wipe 22 to a surface to clean and/or disinfect the surface.

The embodiment shown in Figures 4 and 5 furthermore provides a method of using a wipe 22, the method comprising: storing the wipe 22 in a container 40; storing a cleaning and/or disinfecting compound retained by one or more cyclodextrin compounds in the container 40; storing a fluid comprising water in the container 40, the fluid comprising water being contained in a separate compartment 44 from the cleaning and/or disinfecting compound retained by the one or more cyclodextrin compounds; mixing the fluid comprising water with the cleaning and/or disinfecting compound retained by the one or more cyclodextrin compounds, the one or more cyclodextrin compounds releasing the cleaning and/or disinfecting compound upon being dissolved in the fluid comprising water to produce a solution containing water and the cleaning and/or disinfecting compound; removing the wipe 22 from the container 40, the wipe 22 carrying at least some of the solution containing water and the cleaning and/or disinfecting compound; and applying the wipe 22 to a surface to clean and/or disinfect the surface.

The embodiments shown in Figures 1 to 5 also provide a wipe comprising: a substrate; and an active ingredient retained by one or more cyclodextrin compounds.

The embodiments shown in Figures 1 to 5 also provide a wipe comprising: a substrate; and an alcohol powder.

The embodiments shown in Figures 1 to 5 also provide a method of using the aforementioned wipe, the method comprising: wetting the wipe with one or more fluids comprising water to dissolve the alcohol powder and thereby produce a solution comprising alcohol; and applying the wipe to a surface to deposit at least some of the solution comprising alcohol onto the surface.

It will be understood that, although various features of the invention have been described with respect to one or another of the embodiments of the invention, the various features and embodiments of the invention may be combined or used in conjunction with other features and embodiments of the invention as described and illustrated herein.

The invention is not limited to the specific construction of the wipe 22 or the package 20, 40 that has been shown in the Figures. Rather, any desired shape, size, and material properties of the wipe 22 could be selected as desired. The wipe 22 could also be stored and/or shipped and/or sold in a package 20, 40 that contains multiple wipes 22 and/or which is re-sealable once opened.

Nor is the invention limited to the particular fluids and chemical components of the preferred embodiments. For example, the cleaning and/or disinfecting compound could optionally be retained in a dry form by a chemical component other than cyclodextrin. Optionally, an alcohol powder such as that described in U.S. Patent No. 3,795,747 to Mitchell et al., issued March 5, 1974, which is incorporated herein by reference, could be used in place of or together with the cleaning and/or disinfecting compound retained by the one or more cyclodextrin compounds described above.

The wipes 22 could be adapted for use for a variety of different purposes. For example, the wipes 22 could be hand cleaning wipes 22, human skin cleaning wipes 22, and/or infant human skin cleaning wipes 22 that are adapted for cleaning human skin. If the wipes 22 are to be used for cleaning human skin, the substrate is preferably selected to have a soft texture that will not irritate skin. The cleaning and/or disinfecting compound is also preferably selected to have a concentration and chemical composition that will not irritate human skin. The wipes 22 could also, for example, be counter top wipes 22, table top wipes 22, floor wipes 22, wall wipes 22, furniture wipes 22, and/or instrument or apparatus wipes 22 that are adapted for cleaning the surface or surfaces of one or more different types of objects. If the wipes 22 are to be used for cleaning an object, the substrate is preferably selected to have properties that are suitable for cleaning that object. For example, if the object is prone to scratching, the substrate is preferably selected to have a soft texture that will not scratch the object. Alternatively, if the object has caked on dirt or grease that may be difficult to remove, the substrate may be selected to have an abrasive texture to assist in removing the dirt or grease.

While the invention has been described with reference to specific examples and embodiments, many modifications and variations many occur to persons skilled in the art.

Although this disclosure has described and illustrated certain preferred embodiments of the invention, it is to be understood that the invention is not restricted to these particular embodiments. Rather, the invention includes all embodiments which are functional, mechanical, or chemical equivalents of the specific embodiments and features that have been described and illustrated herein.

## Claims

1. A wipe (22) comprising:
a substrate; and
ethanol retained by one or more cyclodextrin compounds;
wherein the one or more cyclodextrin compounds release the ethanol upon wetting the wipe (22) with water; and
wherein the substrate carries or is impregnated with the ethanol retained by the one or more cyclodextrin compounds.

2. The wipe (22) according to claim 1, wherein the ethanol retained by the one or more cyclodextrin compounds is in a dry powdered form.

3. The wipe (22) according to claim 1 or claim 2, wherein the wipe (22) is stored in a dry state.

4. The wipe (22) according to any one of claims 1 to 3, wherein the substrate is capable of absorbing a fluid.

5. The wipe (22) according to claim 4, wherein the substrate has at least one of: holes, pores, and openings for absorbing the fluid.

6. The wipe (22) according to any one of claims 1 to 5, wherein the substrate comprises at least one of: a sheet, a paper towel-like member, a woven fabric, a mat of bonded fibers, and a porous sponge-like member.

7. A method of using a wipe (22) as claimed in any one of claims 1 to 6, the method comprising:
wetting the wipe (22) with one or more fluids comprising water to release the ethanol from the one or more cyclodextrin compounds; and
applying the wipe (22) to a surface to clean and/or disinfect the surface.

8. The method according to claim 7, wherein the one or more fluids comprising water includes a surface fluid;
wherein, before the wipe (22) is applied to the surface, the surface fluid is present on the surface; and
wherein wetting the wipe (22) comprises applying the wipe (22) to the surface fluid that is present on the surface.

9. The method according to claim 8, further comprising applying the surface fluid to the surface.

10. The method according to claim 8 or claim 9, further comprising drying the surface with the wipe (22);
wherein drying the surface comprises absorbing the surface fluid into the substrate.

11. The method according to any one of claims 7 to 10, wherein applying the wipe (22) to the surface comprises engaging the ethanol with the surface by movement of the wipe (22) in engagement with the surface.

12. The method according to any one of claims 7 to 11, further comprising storing the wipe (22) in a container (20, 40);
wherein the one or more fluids comprising water includes a container fluid; and
wherein wetting the wipe (22) comprises adding the container fluid to the container (20, 40).

13. The method according to claim 12, wherein storing the wipe (22) in the container (20, 40) comprises storing the wipe (22) in the container (20, 40) for a first period of time, before wetting the wipe (22) with the one or more fluids comprising water;
the method further comprising storing the wipe (22) in the container (20, 40) for a second period of time, after the container fluid is added to the container (20, 40).

14. The method according to any one of claims 7 to 13, wherein the one or more fluids comprising water includes a soaking fluid; and
wherein wetting the wipe (22) comprises soaking the wipe in a volume of the soaking fluid to produce, upon release of the ethanol from the one or more cyclodextrin compounds, a solution containing water and ethanol.

15. The method according to claim 14, wherein the solution containing water and ethanol contains at least one of:
at least 10% ethanol by volume;
10% to 70% ethanol by volume;
10% to 40% ethanol by volume; and
no more than 85% ethanol by volume.
